# EUROPÄISCHE PATENTSCHRIFT

(11) **EP 1 214 093 B1**
(45) Veröffentlichungstag und Bekanntmachung des Hinweises auf die Patenterteilung: **01.12.2004**
(21) Anmeldenummer: 00965956.6
(22) Anmeldetag: 09.09.2000
(51) Int. Cl.: A61K 47/08, A61K 47/26

(54) **VERFAHREN ZUR HERSTELLUNG EINES KOMPRIMATES AUS ISOMALTULOSE, ISOMALT ODER ISOMALT-VARIANTEN SOWIE ENTSPRECHENDE PRODUKTE**
METHOD FOR PRODUCING A TABLET MADE OF ISOMALTULOSE, ISOMALT OR ISOMALT VARIANTS AND CORRESPONDING PRODUCTS
PROCEDE DE PREPARATION DE COMPRIMES COMPRENANT L'ISOMALTULOSE, ISOMALT OU SES DERIVES ET PRODUITS SUSCEPTIBLES D'ETRE OBTENUS SELON CE PROCEDE

(30) Priorität: 10.09.1999 DE 19943491
(43) Veröffentlichungstag der Anmeldung: 19.06.2002
(73) Patentinhaber: SÜDZUCKER AKTIENGESELLSCHAFT MANNHEIM/OCHSENFURT, 68165 Mannheim (DE)
(72) Erfinder: BAYERKÖHLER, Theodor, 68165 Mannheim (DE); DÖRR, Tillmann, 67591 Hohen-Sülzen (DE); KOWALCZYK, Jörg, 67248 Bockenheim (DE); KUNZ, Markwart, 67550 Worms (DE); RIFFEL, Peter, 55130 Mainz (DE)
(74) Vertreter: Schrell, Andreas, Dr.
(86) Internationale Anmeldenummer: PCT/EP2000/008832
(87) Internationale Veröffentlichungsnummer: WO 2001/019401

(56) Entgegenhaltungen:
- WO-A-98/37769
- US-A- 5 709 895

## Beschreibung

Die vorliegende Erfindung betrifft ein Verfahren zur Herstellung eines Komprimates aus Isomaltulose, Isomalt und 1,6-GPS- und 1,1-GPM- haltigen Gemischen, die sich durch von Isomalt abweichenden Mengeverhältnissen von 1,1-GPM zu 1,6-GPS auszeichnen und/oder weitere Zuckeralkohole enthalten, sowie die mittels dieses Verfahrens hergestellten Komprimate.

Komprimate sind aus zusammengepreßten Bestandteilen bestehende Genuß-, Arznei- oder auch Nahrungsmittel. Komprimate enthalten dementsprechend im allgemeinen ein Träger- oder Verdünnungsmittel, Bindemittel, Trenn- oder Gleitmittel sowie die aktiven Wirkstoffe, wie Geschmacksstoffe, Arzneistoffe oder Süßungsmittel. Als Träger- beziehungsweise Verdünnungsmittel wird häufig Saccharose, Lactose, Glucose, Stärke oder Mannit verwendet.

Die EP-B1 0 028 905 offenbart Isomaltulose enthaltende Tabletten und Verfahren zu deren Herstellung. Die Druckschrift offenbart eine vorteilhafte Verwendung von Isomaltulose als Verdünnungsmittel für die Komprimatherstellung, da Isomaltulose direkt ohne Bindemittel und ohne kontrollierte Granulierung verpreßt werden könne. Gemäß dieser Druckschrift wird direkt aus der enzymatischen Umwandlung von Saccharose zu Isomaltulose hergestellte kristallisierte Isomaltulose für die Tablettierung verwendet.

Die DE 196 39 343 C2 offenbart Isomalt und Isomalt-Varianten enthaltende Komprimate. Die Herstellung der Komprimate erfolgte durch einfaches Verpressen der Einzelkomponenten ohne eine spezielle mechanische und/oder chemische Behandlung der Einzelkomponenten vorzusehen.

Aus der EP-A1 0 625 578 gehen Isomalt-Varianten hervor, jedoch keine Komprimate, die diese Süßungsmittel enthalten.

Die US 5,709,895 betrifft ein Verfahren zur Herstellung einer Geschmacksstoffe enthaltenden zuckerfreien Kapsel, wobei der Geschmacksstoff in ein Kohlenhydratgemisch aus Polysacchariden und hydrierten Sacchariden eingekapselt wird. Beispielsweise werden Stärke, Dextrin, Isomalt, Zuckeralkohole etc. in deinionisiertem Wasser gelöst, das Gemisch anschließend erhitzt und in einem Kühltank extrudiert. Das Extrudat wird anschließend in Stücke geschnitten und getrocknet.

Die WO 98/37769 betrifft Knusper-Kaugummis, wobei die Knusprigkeit durch den Zusatz von granuliertem Zucker bewirkt wird. Dabei wird ein Verfahren zur Herstellung von granuliertem Isomalt, welches dem Kaugummi zugesetzt wird, beschrieben, wobei Isomalt in Wasser gelöst, auf mindestens 130°C erhitzt, anschließend abgekühlt und als Feststoff granuliert wird.

Die im Stand der Technik bekannten Isomaltulose-, Isomalt- und Isomalt-Varianten-haltigen Komprimate zeichnen sich allesamt durch die Notwendigkeit aus, vergleichsweise hohe Preßdrücke bei der Komprimat-Herstellung einzusetzen, wobei vergleichsweise nur geringe Tablettenhärten erzielt werden können. Zudem sind die Komprimate des Standes der Technik hinsichtlich ihrer sensorischen Eigenschaften verbesserungsfähig, zeigen zum Beispiel Rauhigkeit beim Zerbeißen sowie nachteiliges Bruchverhalten und ein verbesserungsfähiges Ablutschverhalten.

Das der vorliegenden Erfindung zugrundeliegende technische Problem besteht also darin, ein Verfahren zur Herstellung von Komprimaten aus Isomaltulose, Isomalt oder 1,6-GPS- und 1,1-GPM-haltigen Gemischen, die sich durch von Isomalt abweichende Mengenverhältnisse von 1,1-GPM zu 1,6-GPS auszeichnen und/oder weitere Zuckeralkohole enthalten, bereitzustellen, welches die vorgenannten Nachteile beseitigt, insbesondere unter Verwendung möglichst niedriger Preßdrücke Komprimate großer Härte herzustellen, die sich durch verbesserte sensorische Eigenschaften und ein verbessertes Bruchverhalten auszeichnen.

Die vorliegende Erfindung löst das ihr zugrundeliegende technische Problem durch die Bereitstellung eines Verfahrens nach Anspruch 1 zur Herstellung eines Komprimates aus Isomaltulose, Isomalt oder 1,6-GPS- und 1,1-GPM-haltigen Gemischen, die sich durch von Isomalt abweichende Mengenverhältnisse von 1,1-GPM zu 1,6-GPS auszeichnen und/oder weitere Zuckeralkohole enthalten, wobei in einem ersten Verfahrensschritt die Isomaltulose, das Isomalt und/oder das 1,6-GPSund 1,1-GPM-haltige Gemisch trocken gemahlen, in einem zweiten Verfahrensschritt gleichzeitig oder nach dem ersten Verfahrensschritt eine gemahlene Fraktion Isomaltulose, Isomalt oder das 1,6-GPS und 1,1-GPM-haltige Gemisch mit einem Partikeldurchmesser d₉₀ von maximal 100 µm (d₉₀ = 90 % der Partikel mit erforderlichem Durchmesser) erhalten oder abgetrennt, in einem dritten Verfahrensschritt die abgetrennte gemahlene Fraktion unter Zusatz eines flüssigen Bindemittels agglomeriert und anschließend in einem vierten Verfahrensschritt das Agglomerat zu einem Komprimat verpreßt wird. Die Erfindung löst das ihr zugrundeliegende technische Problem auch durch die Bereitstellung eines gemäß des vorliegenden Verfahrens hergestellten Komprimates nach Anspruch und Agglomerats nach Anspruch 17.

Die Erfindung sieht also vor, daß aus einem oder mehreren der Edukte Isomaltulose, Isomalt oder 1,6-GPS- und 1,1-GPM-haltige Gemische, die sich durch von Isomalt abweichende Mengeverhältnisse von 1,1-GPM zu 1,6-GPS auszeichnen und/oder weitere Zuckeralkohole enthalten, ein Komprimat hergestellt wird, indem eines oder mehrerer der Edukte trocken gemahlen wird, wobei entweder nach oder während des Mahlens eine Fraktion abgetrennt und gewonnen wird, deren Partikelgröße maximal 100 µm beträgt. Die erfindungsgemäße Einstellung der Primärkornverteilung erweist sich dabei als von großer Bedeutung. Das Vermahlen wird vorzugsweise in einer Sichtermühle beziehungsweise einer Kombination von Mühle und nachgeschaltetem Sichter durchgeführt. Die erhaltene Fraktion wird unter Zusatz eines flüssigen Bindemittels agglomeriert und kann anschließend nach Zugabe weiterer Komponenten, wie zum Beispiel Aromen, Preßhilfsmittel, und andere, direkt verpreßt werden. Mittels des erfindungsgemäßen Verfahrens können preßfertige Mischungen hergestellt werden, die sich zu Komprimaten verarbeiten lassen, die zu einer vorteilhaften erheblichen Reduktion der Preßkraft führen und die daraus resultierenden Komprimate gleichzeitig eine sehr gute Bruchkraft bei gleichzeitig verbesserten sensorischen Eigenschaften aufweisen. Die erfindungsgemäße Verfahrensweise ermöglicht es also, deutlich niedrigere Preßkräfte als im Stand der Technik zu verwenden, um ausreichend harte Tabletten zu erhalten. Die direkt verpreßbaren Mischungen zeigen eine verbesserte Fließfähigkeit und einen geringeren Staubanteil. Dieses führt zu einer besseren Verarbeitbarkeit und einer Verringerung des Maschinenverschleißes sowie einer Erhöhung der Tablettierleistung.

Die Agglomerate zeichnen sich im allgemeinen durch eine gute Fließfähigkeit und gute selbstbindende Eigenschaften aus, die ein Verkleben an einer Presse weitgehend oder ganz unmöglich machen. Im Zusammenhang mit der vorliegenden Erfindung wird unter einem Komprimat ein aus zusammengepreßten Bestandteilen bestehendes Genuß-, Arznei- oder Nahrungsmittel verstanden. Ein Komprimat im Sinne der vorliegenden Erfindung ist zum Beispiel eine Tablette. Komprimate können Hilfs- und Zusatzstoffe, wie Schmiermittel, Binder, Verdünnungsmittel sowie Aromastoffe, Geschmacksstoffe, Trennmittel, Farbstoffe, Säuerungsmittel, Vitamine, Functional Foods, Süßungsmittel und/oder Arzneistoffe enthalten.

Im Zusammenhang mit der vorliegenden Erfindung wird unter Isomalt ein auch unter dem Namen Palatinit® bekanntes nahezu äquimolares Gemisch der beiden Stereoisomere 6-O-α-D-Glucopyranosyl-D-sorbit (1,6-GPS) und 1-O-α-D-Glucopyranosyl-D-mannit (1,1-GPM) verstanden. 1,6-GPS- und 1,1-GPM-haltige Gemische, die sich beispielsweise durch von Isomalt abweichende Mengenverhältnisse von 1,1-GPM zu 1,6-GPS auszeichnen und/oder weitere Zuckeralkohole wie 1,1-GPS (1-O-α-D-Glucopyranosyl-D-sorbit) enthalten, und die auch als Isomalt-Varianten bezeichnet werden, gehen beispielsweise aus der EP-A1 0 625 578 hervor, die hinsichtlich der quantitativen und qualitativen Zusammensetzung der 1,1-GPM- und 1,6-GPS-haltigen Zuckeralkoholgemische und Verfahren zu deren Herstellung in den Offenbarungsgehalt der vorliegenden Anmeldung mit einbezogen ist. Solche Gemische können daher beispielsweise Gemische aus 10 bis 50 Gew.-% 1,6-GPS, 2 bis 20 Gew.-% 1,1-GPS und 30 bis 70 Gew.-% 1,1-GPM oder Gemische aus 5 bis 10 Gew.-% 1,6-GPS, 30 bis 40 Gew.-% 1,1-GPS und 45 bis 60 Gew.-% 1,1-GPM darstellen. Gemäß des Vorstehenden können solche Gemische auch 1,6-GPS- oder 1,1-GPM-angereicherte Gemische darstellen, also Gemische, wie sie in der DE 195 32 396 C2 beschrieben sind, die hinsichtlich der quantitativen und qualitativen Zusammensetzung der dort beschriebenen Gemische und Verfahren zu deren Herstellung in den Offenbarungsgehalt der vorliegenden Anmeldung mit einbezogen ist. 1,6-GPS-angereicherte Gemische zeichnen sich durch einen 1,6-GPS-Anteil von 57 bis 99 Gew.-% und einen 1,1-GPM-Anteil von 43 bis 1 Gew.-% aus, während sich 1,1-GPM-haltige Gemische durch einen 1,6-GPS-Anteil von 1 bis 43 Gew.-% und einen 1,1-GPM-Anteil von 57 bis 99 Gew.-% auszeichnen. Selbstverständlich können die vorgenannten 1,6-GPS- und 1,1-GPM-haltigen Gemische oder das Isomalt weitere Stoffe wie Mannit, Sorbit, hydrierte oder nichthydrierte Oligosaccharide sowie gegebenenfalls Glucose, Fructose und/oder Saccharose, Trehalulose oder Isomaltose enthalten.

Die Erfindung sieht also vor, daß nach einem ersten Verfahrensschritt das Edukt, nämlich Isomaltulose, Isomalt oder/und die 1,6-GPS und 1,1-GPM-haltigen Gemische trocken gemahlen wird. Dies kann in bevorzugter Ausführungsform der Erfindung in einer Sichtermühle beziehungsweise Kombination von Mühle und nachgeschaltetem Sichter geschehen. Selbstverständlich sieht die Erfindung auch vor, die eingesetzten Edukte auf die erforderliche Partikelgröße mittels anderer Maßnahmen als Vermahlen zu bringen, beispielsweise durch Zerstampfen. Beim Vermahlen können bereits Hilfs- und Zusatzstoffe hinzugegeben werden, bevorzugt in einer Menge bis zu 30 Gew.-% (bezogen auf Gesamttrockensubstanz).

Die Erfindung sieht in einem im wesentlichen gleichzeitig oder anschließend an das Vermahlen stattfindenden zweiten Verfahrensschritt vor, eine Fraktion abzutrennen und der Weiterverwendung durchzuführen, wobei die darin enthaltenen Partikel eine Maximalgröße von 100 µm, bevorzugt kleiner als 50 µm, insbesondere eine Maximalgröße von 40 µm, bevorzugt 35 µm und besonders bevorzugt 30 µm aufweisen. Sofern nach dem ersten Verfahrensschritt, also dem Vermahlen, das eingesetzte Edukt bereits vollständig die erforderliche Partikelgröße d₉₀ (d₉₀ = 90 % der Partikel mit erforderlichem Durchmesser) aufweist, kann auf die Abtrennung verzichtet und die erhaltene Pulver direkt dem dritten Verfahrensschritt zugeführt werden. Das Vermahlen und Abtrennen kann selbstverständlich gleichzeitig, zum Beispiel in einer Sichtermühle beziehungsweise einer Kombination von Mühle und nachgeschaltetem Sichter, erfolgen.

Im Zusammenhang mit der vorliegenden Erfindung wird unter einem Partikeldurchmesser von maximal 100 µm, 40 µm, 35 µm oder 30 µm verstanden, daß mindestens 90 % der Partikel (d₉₀) der gemahlenen Fraktion einen Durchmesser von maximal 100 µm, 40 µm, 35 µm oder 30 µm aufweisen.

In einem dritten Verfahrensschritt wird erfindungsgemäß vorgesehen, der abgetrennten gemahlenen Fraktion ein flüssiges Bindemittel zuzusetzen. In einer besonders bevorzugten Ausführungsform der Erfindung ist dieses flüssige Bindemittel eine, insbesondere wässrige, Lösung oder Suspension von Isomalt, einer Isomalt-Variante, ein Gemisch aus Gelatine und Fett, ein wasserlösliches Kolloid, wie Polyvinylpyrrolidon (zum Beispiel Kollidon® der Firma BASF), Stärke, Zucker wie Saccharose, Dextrose, Lactose, natürliche oder synthetische Gummis wie Gummi arabicum, Cellulose, Talkum, mikrokristalline Cellulose, polymerisierte reduzierende Zucker, Pektin, Konservierungsmittel, Agar Agar, Säuerungsmittel, Inulin, Alkalicarboxymethylcellulose, HSH (hydrierte Stärkehydrolysate), Polydextrose in teilweise oder vollständig gereinigter und/oder in teilweise oder vollständig neutralisierter Form, Natriumcarboxymethylcellulose etc. Selbstverständlich sind auch andere Bindemittel einsetzbar, wobei es bevorzugt ist, physiologisch verträgliche und/oder nicht kariogene, brennwertreduzierte Bindemittel einzusetzen. In vorteilhafter Weise enthält das erfindungsgemäße Komprimat 0,5 bis 7 Gew.-% des Bindemittels beziehungsweise eine Kombination von Bindemitteln, vorzugsweise 2 bis 3 Gew.-%.

In einer weiteren bevorzugten Ausführungsform der Erfindung ist vorgesehen, daß das flüssige, also bevorzugt in Form einer wässrigen Lösung oder wässrigen Suspension vorliegende Bindemittel dem vermahlenen Edukt durch Sprühen über ein Düsensystem zugeführt wird.

Die Herstellung der sich nach dem Mischen des E-dukts mit dem Bindemittel bildenden Agglomerate kann vorzugsweise in einem Wirbelschichtagglomerator, besonders bevorzugt in batchweiser Verfahrensdurchführung beziehungsweise in einer kontinuierlich arbeitenden Anlage, durchgeführt werden. Dabei ist es erfindungsgemäß bevorzugt, ein Wirbelbett bei einer Temperatur von 50° bis 70°C, insbesondere 60°C aufzubauen und bei Erreichen der gewünschten Temperatur auf circa 70° bis 80°C, vorzugsweise 75°C, also über Raumtemperatur erhitzte Bindemittel-Lösung oder Bindemittel-Suspension in das Wirbelbett einzusprühen. Die Temperatur des Bindemittels ist je nach eingesetztem Bindemittel so zu wählen, daß das Bindemittel sprühfähig ist, das heißt, daß die Temperatur bei oder über dem Schmelzpunkt des Bindemittels liegt. Im Anschluß an die Agglomeration kann in einer weiteren bevorzugten Ausführungsform der Erfindung vorgesehen sein, eine Trocknung durchzuführen, die in einer weiteren bevorzugten Ausführungsform bei konstanter Zulufttemperatur, zum Beispiel von 70° bis 90°C, besonders bevorzugt 80°C, durchgeführt wird. In einer weiteren bevorzugten Ausführungsform kann vorgesehen sein, die Trocknung bis zu einer Ablufttemperatur von 50° bis 70°C, vorzugsweise 60°C, durchzuführen, wobei die Produktabkühlung vorzugsweise mit Außenluft geschieht.

Die Erfindung betrifft selbstverständlich auch die wie vorstehend hergestellten Agglomerate selbst.

Die Erfindung sieht in einer weiteren vorteilhaften Ausgestaltung der vorliegenden Lehre vor, nach der Zugabe des Bindemittels und dem Agglomerieren, aber vor dem Verpressen des Agglomerats, eine Größenfraktionierung, insbesondere eine Abtrennung von Überkorn und Stäuben, der agglomerierten Produkte durchzuführen. Dabei kann vorzugsweise eine Siebmaschine mit einer Siebbelegung von beispielsweise 0,8 mm bis 0,1 mm vorgesehen sein.

In einem vierten Verfahrensschritt ist erfindungsgemäß vorgesehen, das agglomerierte und gegebenenfalls nach der Agglomeration fraktionierte Produkt direkt zu verpressen. Dabei kann vorgesehen sein, den Agglomeraten Hilfs- oder Zusatzstoffe, wie Trenn- oder Gleitmittel, Wirkstoffe etc., hinzuzufügen. Derartige Stoffe können Süßstoffe, Aromen, Geschmacks- und Farbstoffe, lebensmittelverträgliche Säuren, Sprengmittel, Monosaccharide, Disaccharide, Monosaccharidalkohole, Disaccharidalkohole, Stärke, Stärkederivate, Pektin, Polyvinylpyrrolidon, Cellulose, Cellulosederivate, Stearinsäure oder deren Salze oder Inulin, Oligofructose beziehungsweise andere Produkte, zum Beispiel Functional Foods, die entsprechend ausgelobt werden können, sein. Den Agglomeraten können auch Sorbit, Mannit, hydrierte oder nicht-hydrierte Oligosaccharide, Erythrit, Xylit oder Zucker, wie Saccharose, Glucose, Lactose, Fructose oder Xylose, zugefügt werden. In vorteilhafter Weise liegt der Anteil dieser Stoffe, bezogen auf das Gesamttrockengewicht, bei einer Menge von gleich oder weniger als 30 Gew.-%, vorzugsweise weniger als 25, 20, 15, 10 oder 5 Gew.-%. Die genannten Hilfs- und Zusatzstoffe können selbstverständlich den Edukten auch schon beim Vermahlen zugefügt werden. Die vorstehend genannten Komponenten wie Hilfs- oder Zusatzstoffe, zum Beispiel Erythrit, können also in einer weiteren Ausführungsform der vorliegenden Erfindung zusammen mit der Isomaltulose, dem Isomalt und/oder der Isomalt-Variante zusammen trocken gemahlen werden und anschließend erfindungsgemäß weiter behandelt werden. In einer anderen Ausgestaltung der vorliegenden Erfindung können die vorgenannten Zusatz- oder Hilfsstoffe, wie Erythrit, in einem Lösungsmittel, zum Beispiel Wasser, gelöst und während des Agglomerierens der gemahlenen Isomaltulose, des Isomalts und/oder der Isomalt-Variante in diese gemahlene Fraktion gesprüht werden, so daß das Einbringen der gelösten Zusatz- oder Hilfsstoffe während des Agglomerationsprozesses geschieht. Schließlich kann in einer dritten Ausgestaltung der Erfindung vorgesehen sein, die genannten Zusatz- oder Hilfsstoffe zusammen mit der agglomerierten Fraktion zu mischen und zu einem Komprimat zu verpressen.

In besonders vorteilhafter Ausführungsform sind die erfindungsgemäß hergestellten Komprimate zuckerfrei. Die Komprimate oder Agglomerate können auch in einer Ausführungsform Xylit-frei sein. In einer weiteren bevorzugten Ausführungsform sind die erfindungsgemäßen Komprimate brennwertreduziert, Diabetiker-geeignet, Blutfett-reduzierend, bifidogen und/oder zahnfreundlich.

Den Agglomeraten oder Edukten können ferner Intensiv-Süßstoffe, wie Dipeptid-Süßstoffe, Saccharin, Acesulfam-K, Aspartam, Cyclamat, Glycyrrhizin, Taumatin, Saccharin, Steveoside, Neohesperidin-Dihydrochalkon und/oder Sucralose zugefügt werden.

In vorteilhafter Weise enthalten die erfindungsgemäßen Komprimate zudem Geschmacks- oder Aromastoffe, wie Citronen- oder Pfefferminz-Aroma. Die erfindungsgemäßen Komprimate können auch lebensmittelverträgliche Säuren, wie Ascorbinsäure oder Citronensäure, sowie als Gleitmittel Fettsäuren oder deren Salze, wie Magnesiumstearat oder Natriumstearat, enthalten. Schließlich kann vorgesehen sein, daß in den erfindungsgemäßen Komprimaten Farbstoffe und/oder Sprengmittel, wie Bicarbonat oder Carboxymethylcellulose, enthalten sind.

In einer besonders bevorzugten Ausführungsform ist vorgesehen, Komprimate bereitzustellen, die pharmazeutisch aktive Wirkstoffe in den Mund- und Rachenraum bringen und dort freisetzen können. Im Zusammenhang mit der vorliegenden Erfindung sind unter pharmazeutisch aktiven Wirkstoffen Substanzen zu verstehen, die einen erwünschten prophylaktischen oder therapeutischen Effekt auf den menschlichen oder tierischen Körper haben. Diese Substanzen dienen also insbesondere der Prophylaxe oder Therapie von Mangelzuständen oder Krankheitsbildern. Erfindungsgemäß können beispielsweise Enzyme, Coenzyme, Mineralstoffe, Vitamine, Antibiotica, microbizid oder fungizid wirkende Stoffe, Nikotin, Coffein, Zink, Eukalyptus, Menthol, Codein, Phenacetin, Acetylsalicylsäure oder andere pharmazeutisch aktive Stoffe in die Komprimate eingeschlossen werden. Die pharmazeutisch aktiven Wirkstoffe sind in einer Menge vorzusehen, die den erwünschten pharmazeutischen Effekt bewirken. Die schonende Verarbeitbarkeit der Komprimate machen die erfindungsgemäßen Komprimate besonders geeignet, pharmazeutisch aktive Wirkstoffe in den Mund- und Rachenraum zu verbringen.

Die Erfindung betrifft auch die mittels der vorgenannten Verfahren hergestellten Komprimate, insbesondere in Form von Lutsch-, Kau- oder Brausetabletten.

Weitere vorteilhafte Ausgestaltungen der Erfindung ergeben sich aus den Unteransprüchen.

Die folgenden Beispiele erläutern die Erfindung in Einzelheiten.

Die Figuren zeigen:
- Figur 1: einen Bruchkraftvergleich zwischen Komprimaten aus Isomalt ST (Typ FE, nicht agglomeriert), Isomalt ST (agglomeriert) und Isomalt GS (agglomeriert),
- Figur 2: einen Bruchkraftvergleich zwischen Komprimaten aus Isomalt ST und Isomalt GS und
- Figur 3: Bruchkraftangaben zu Isomaltulose-haltigen Komprimaten.

### Beispiel 1: Herstellung von Komprimaten

Isomalt ST (Standard, nahezu äquimolares Gemisch aus 1,1-GPM und 1,6-GPS) wurde trocken bis zu einer d₉₀ = 50 µm in einer Sichtermühle vermahlen. Ebenso wurde mit Isomalt GS (Zusammensetzung circa 76 % 1,6-GPS und 23 % 1,1-GPM) und Isomaltulose verfahren. Isomalt ST Typ FE (wie Isomalt ST, aber nicht agglomiert; 60 bis 300 µm Partikelgröße) diente als Kontrolle.

Zur Herstellung der Agglomerate wurde ein Wirbelschichtagglomerator im Batch-Verfahren verwendet, und zwar der Agglomerator STREA 7 der Firma Aeromatic. Die Versuchschargen betrugen jeweils 10 kg. Dabei wurde das gemahlene Schüttgut im Wirbelschichtagglomerator vorgelegt und ein Wirbelbett bei circa 60°C aufgebaut. Beim Erreichen dieser Temperatur wurde eine circa 75°C heiße Bindemittellösung in das Wirbelbett eingespritzt, wobei entweder 3 Gew.-% Kollidon 30 oder 0,8 Gew.-% Gelatine (130 Bloom) und 0,5 Gew.-% Fett eingesetzt wurden. Der eingesetzte Sprühdruck betrug zwischen 2,0 und 4,5 bar, wobei ein Vordruck von 0,4 bis 0,8 bar eingesetzt wurde. Anschließend wurden die gebildeten Agglomerate bei konstanter Zulufttemperatur von circa 80°C bis zu einer Ablufttemperatur von circa 60°C getrocknet, wobei die Produktabkühlung mit Außenluft erfolgt. Anschließend wurde mittels einer Taumelsiebmaschine mit einer Siebbelegung 0,8 mm bis 0,1 mm eine Größenfraktionierung durchgeführt. Dabei wurden Überkorn und Stäube abgetrennt. Agglomeratfraktionen mit einem Partikeldurchmesser von ≥ 0,1 mm bis ≤ = 0,8 mm wurden dann weiterverwendet, um nach Zugabe von Aromen, Intensiv-Süßstoffen und Trennmitteln gemäß nachstehender Rezeptur die Komprimate zu pressen.

Rezeptur:

| | |
|---|---|
| Isomalt, Isomalt-Variante (GS) oder Isomaltulose-Agglomerat: | 98,40 % |
| Mg-Stearat: | 0,50 % |
| natürliches Citronenaroma | 0,50 % |
| Citronensäure (Mono.) | 0,30 % |
| Acesulfam-K | 0,15 % |
| Aspartam | 0,15 % |

Alle Angaben in Gew.-%, bezogen auf Gesamttrockengewicht des Komprimats.

Die nachfolgende Tabelle zeigt chemischphysikalische Parameter der eingesetzten Komprimat-Mischungen auf.

| Rezeptur | Wassergehalt | d05 | d95 | d' | n | Schüttdichte | Stampfdichte | Rieselzeit |
|---|---|---|---|---|---|---|---|---|
| | % | mm | mm | mm | | g/cm³ | g/cm³ | s |
| Isomalt ST (K) | 4,1 | 0,53 | 0,07 | 0,31 | 2,01 | 0,44 | 0,45 | 23,0 |
| Isomalt ST(G-F) | 3,9 | 0,53 | 0,06 | 0,3 | 1,88 | 0,51 | 0,52 | 24,7 |
| Isomalt GS (K) | 1,9 | 0,53 | 0,06 | 0,3 | 1,92 | 0,42 | 0,42 | 24,6 |
| Isomalt GS(G-F) | 1,4 | 0,7 | 0,09 | 0,4 | 1,94 | 0,53 | 0,54 | 18,9 |
| Isomaltulose (K) | 5,1 | 0,53 | 0,04 | 0,26 | 1,55 | 0,44 | 0,45 | 22,8 |
| Tabelle (K: Kollidon 30; G-F: Gelatine-Fett) Bestimmung der Rieselfähigkeit und der Rieselzeit nach DIN 53194 und DIN 53492 Art der Düse zur Bestimmung der Rieselfähigkeit: 10 mm Durchmesser Bestimmung der Schütt- und Stampfdichtigkeit nach DIN 53194 | | | | | | | | |

Die vorgenannten Mischungen für die Komprimat-Versuche wurden im Pflugscharmischer der Firma Lödige hergestellt. Die Mischzeit lag bei 1,5 min. Die Zudosierung der Einzelkomponenten erfolgte über eine Öffnung in der Deckelklappe des Mischers. Nach Beendigung des Mischvorganges wurden die Mischungen in PE-Säcken von je 5 kg abgefüllt und verschweißt.

Anschließend wurden mit den so erhaltenen Mischungen mittels einer FETTE PT 2090 Rundläuferpresse runde Tabletten mit einem Durchmesser von 18 mm und Facette, einer Steghöhe von 0,35 bis 0,37 mm und einem Gewicht zwischen 850 mg bis 1000 mg hergestellt.

### Beispiel 2: Bruchkraftvergleiche

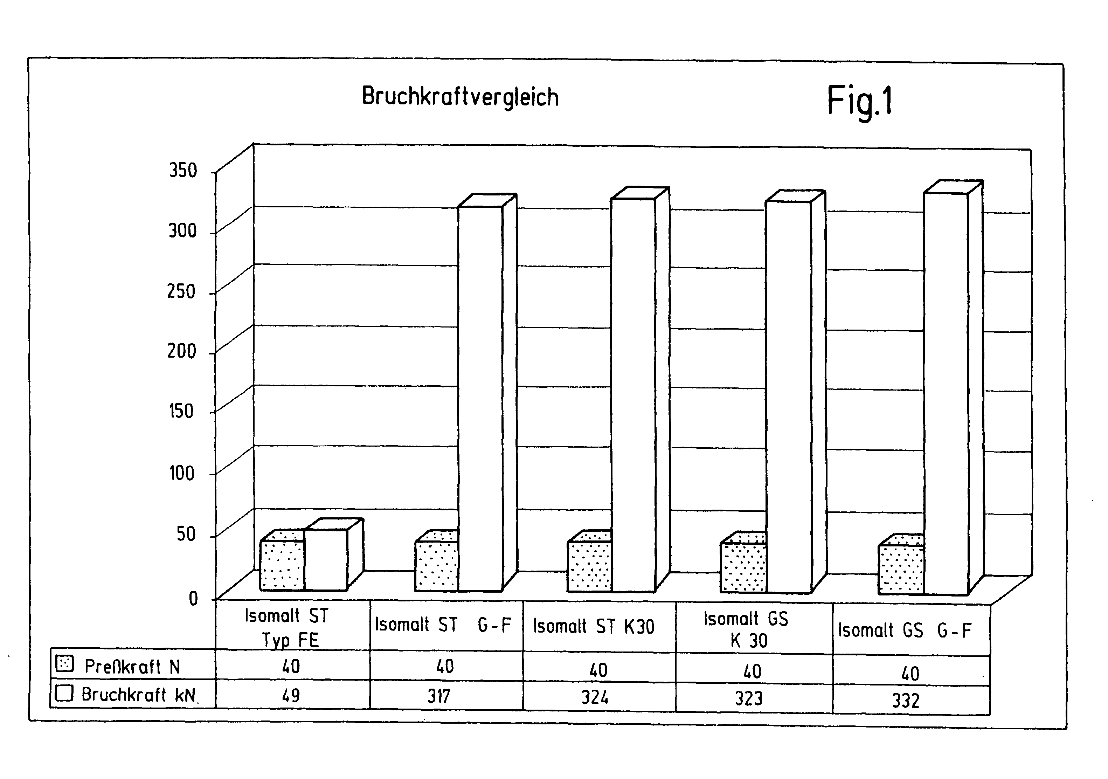

Die Figur 1 zeigt einen Bruchkraftvergleich zwischen Komprimaten aus agglomeriertem Isomalt ST (K und G-F) und agglomeriertem Isomalt GS (K und G-F). Als Vergleich ist auch ein Komprimat aus Isomalt ST Typ FE dargestellt, hergestellt aus einer Fraktion von Partikeln mit einem Partikeldurchmesser von 60 bis 300 µm. Die erfindungsgemäßen Komprimate wurden mit einer Preßkraft von nur 40 kN hergestellt, weisen jedoch eine extrem hohe Bruchkraft auf. (Die Abkürzungen in den Figuren bedeuten: K 30: Kollidon 30; GF: Gelatine-Fett).

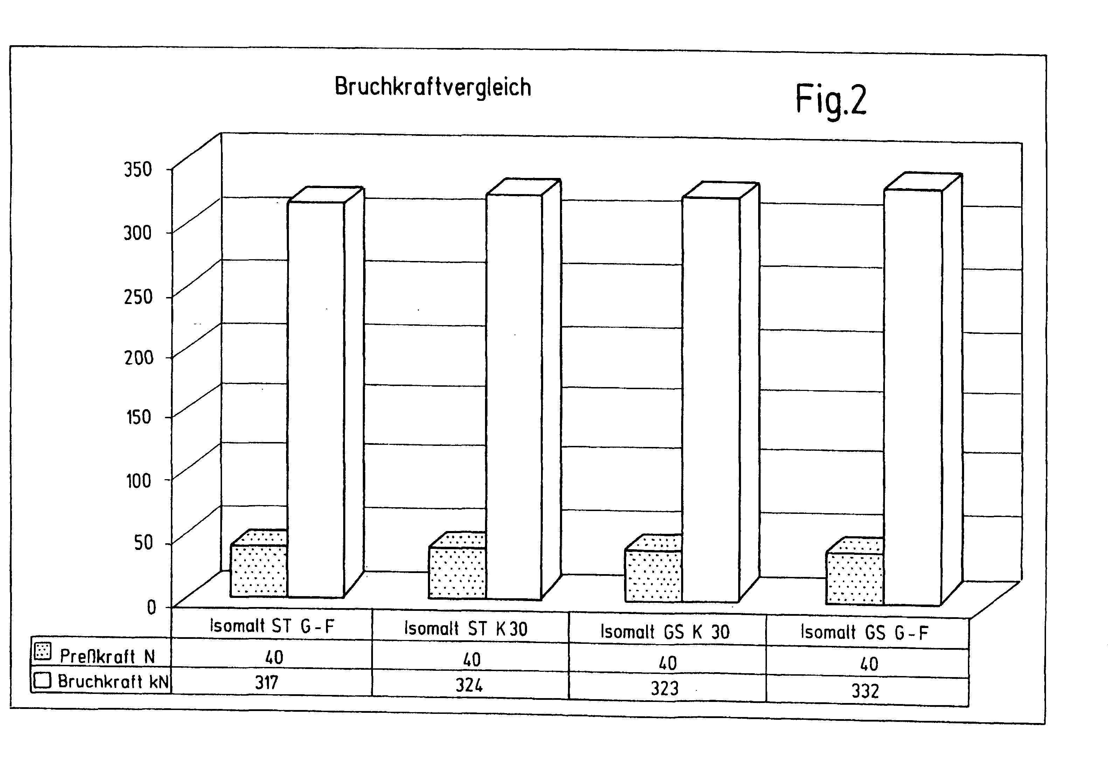

Figur 2 zeigt einen Bruchkraftvergleich zwischen Isomalt ST und Isomalt GS, beide in agglomerierter Form. Es zeigt sich, daß sich zwischen diesen beiden Isomalt-Formen kein signifikanter Unterschied ergibt. Bei einer sensorischen Beurteilung wurde das Ablutschverhalten der GS-Variante als geringfügig besser beurteilt.

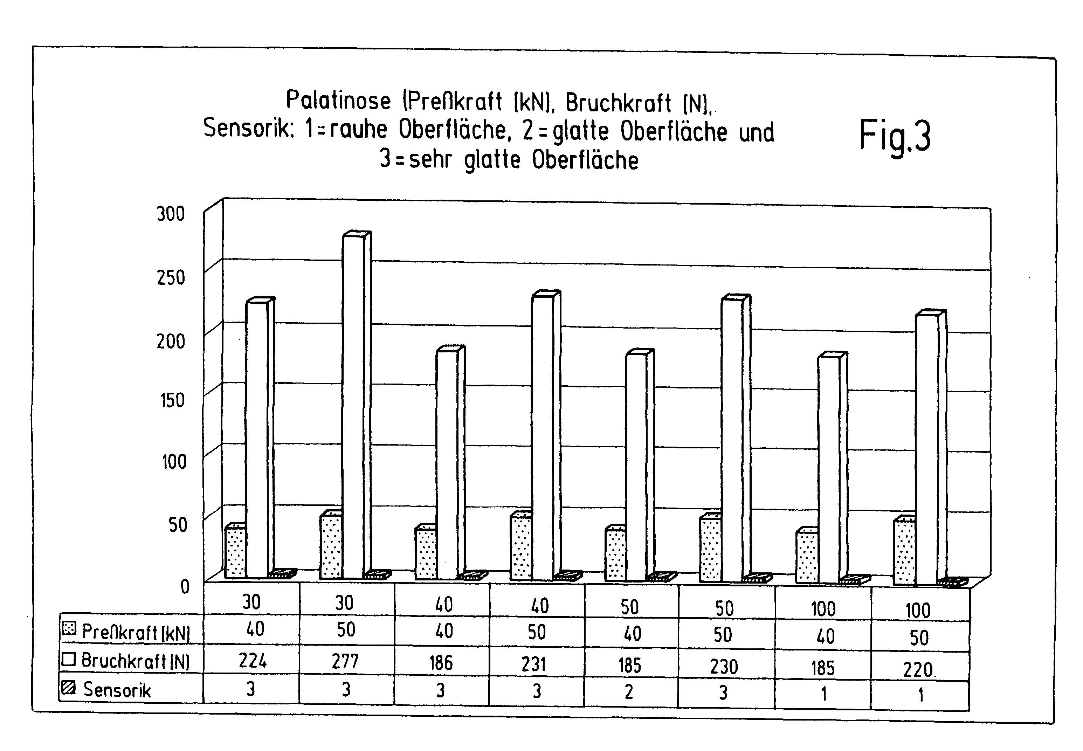

Die Figur 3 vergleicht auf der Basis von Isomaltulose (gleich Palatinose) hergestellte Komprimate. Dargestellt sind Preßkraft, Bruchkraft und Sensorik von Isomaltulose-Komprimaten, hergestellt aus einer Fraktion mit einer Partikelgröße von 100 µm und Kollidon 30. Die Verwendung einer Fraktion mit Partikeln eines Durchmessers ≤ 100 µm, bevorzugt ≤ 50 µm, insbesondere ≤ 30 µm, ist bei Isomaltulose besonders wichtig, da Isomaltulose-Fraktionen mit Partikelgrößen > 100 µm beim Komprimieren stark zu einer spürbar rauhen Oberfläche neigen.

Die vorstehenden Ergebnisse zeigen, daß die erfindungsgemäß vorgegebene Agglomeration dazu führt, daß deutlich niedrigere Preßkräfte als im Stand der Technik verwendet werden können, um Tabletten mit ausreichender Bruchkraft zu erhalten. Die Mischung mit den so hergestellten Agglomeraten führt zu einer verbesserten Fließfähigkeit und einem geringeren Staubanteil, was zu einer besseren Verarbeitbarkeit und zur Verringerung des Maschinen-Verschleisses sowie einer Erhöhung der Tablettierleistung führt.

## Patentansprüche

1. Verfahren zur Herstellung eines Komprimates aus Isomaltulose, Isomalt oder 1,6-GPS- und 1,1-GPM-haltigen Gemischen, die sich durch von Isomalt abweichende Mengenverhältnisse von 1,1-GPM zu 1,6-GPS auszeichnen und/oder weitere Zuckeralkohole enthalten, wobei
a) die Isomaltulose, das Isomalt und/oder das 1,6-GPS- und 1,1 -GPM-haltige Gemisch trocken gemahlen,
b) gleichzeitig oder danach eine gemahlene Fraktion der Isomaltulose, des Isomalts oder des 1,6-GPS- und 1,1-GPM-haltigen Gemisches mit einem Partikeldurchmesser von maximal 100 µm erhalten oder abgetrennt,
c) die gemahlene Fraktion unter Zusatz eines flüssigen Bindemittels agglomeriert und
d) anschließend zu einem Komprimat verpresst wird.

2. Verfahren nach Anspruch 1, wobei das 1,6-GPSund 1,1-GPM-haltige Gemisch ein Gemisch aus 10 bis 50 Gew.-% 1,6-GPS, 2 bis 20 Gew.-% 1,1-GPS und 30 bis 70 Gew.-% 1,1-GPM oder ein Gemisch aus 5 bis 10 Gew.-% 1,6-GPS, 30 bis 40 Gew.-% 1,1-GPS und 45 bis 60 Gew.-% 1,1-GPM oder ein 1,6-GPS angereichertes Gemisch mit einem 1,6-GPS-Anteil von 57 bis 99 Gew.-% und einem 1,1-GPM -Anteil von 43 bis 1 Gew.-% oder ein 1,1-GPM angereichertes Gemisch mit einem 1,6-GPS-Anteil von 1 bis 43 Gew.-% und einem 1,1-GPM-Anteil von 57 bis 99 Gew.-% ist.

3. Verfahren nach Anspruch 1 oder 2, wobei der Partikeldurchmesser ≤ 50 µm beträgt.

4. Verfahren nach Anspruch 1, 2 oder 3, wobei der partikeldurchmesser ≤ 30 µm beträgt.

5. Verfahren nach einem der vorhergehenden Ansprüche, wobei die Vermahlung in einer Sichtermühle oder in einer Kombination von Mühle und nachgeschaltetem Sichter erfolgt.

6. Verfahren nach einem der vorhergehenden Ansprüche, wobei beim Vermahlen Hilfs- oder Zusatzstoffe eingebracht werden.

7. Verfahren nach einem der vorhergehenden Ansprüche, wobei das flüssige Bindemittel eine Lösung oder Suspension von Isomalt, eines 1,6-GPS- und 1,1-GPM-haltigen Gemisches, das sich durch von Isomalt abweichende Mengenverhältnisse von 1,1-GPM zu 1,6-GPS auszeichnet, Fett und Gelatine oder Kollidon ist.

8. Verfahren nach einem der vorhergehenden Ansprüche, wobei das flüssige Bindemittel mittels Sprühen der abgetrennten gemahlenen Fraktion zugeführt wird.

9. Verfahren nach einem der vorhergehenden Ansprüche, wobei das flüssige Bindemittel über eine Düse der abgetrennten gemahlenen Fraktion zugeführt wird.

10. Verfahren nach einem der vorhergehenden Ansprüche, wobei die Agglomeration in einem Wirbelschichtagglomerator satzweise oder in einer kontinuierlich arbeitenden Anlage durchgeführt wird.

11. Verfahren nach einem der vorhergehenden Ansprüche, wobei das flüssige Bindemittel in über Raumtemperatur liegender erhitzter Form der abgetrennten gemahlenen Fraktion zugeführt wird.

12. Verfahren nach einem der vorhergehenden Ansprüche, wobei dem Agglomerat nach Zugabe des flüssigen Bindemittels und vor dem Verpressen Hilfsstoffe und/oder Aromen hinzugefügt werden.

13. Verfahren nach einem der vorhergehenden Ansprüche, wobei nach Zugabe des flüssigen Bindemittels und vor dem Verpressen eine Größenfraktionierung des Agglomerats durchgeführt wird.

14. Verfahren nach einem der vorhergehenden Ansprüche, wobei die Größenfraktionierung des Agglomerats nach Anspruch 13 in einer Siebmaschine durchgeführt wird.

15. Verfahren nach einem der vorhergehenden Ansprüche, wobei im Anschluß an die Agglomeration das Agglomerat getrocknet wird.

16. Komprimat, herstellbar nach einem der vorhergehenden Ansprüche.

17. Agglomerat, herstellbar nach den Verfahrensschritten a) bis c) nach einem der Ansprüche 1 bis 15.

## Claims

1. Method of producing a compressed product of isomaltulose, isomalt or mixtures containing 1,6-GPS and 1,1-GPM, which are **characterized by** quantity ratios of 1,1-GPM to 1,6-GPS which differ from those of isomalt and/or contain other sugar alcohols, wherein
a) the isomaltulose, isomalt and/or the mixture containing 1,6-GPS and 1,1-GPM is ground dry,
b) at the same time or thereafter, a ground fraction of the isomaltulose, the isomalt or the mixture containing 1,6-GPS and 1,1-GPM with a maximum particle diameter of 100 µm is obtained or separated,
c) the ground fraction is agglomerated with the addition of a liquid binder, and
d) then it is compressed to form a compressed product.

2. Method according to claim 1, wherein the mixture containing 1,6-GPS and 1,1-GPM is a mixture of 10 wt% to 50 wt% 1,6-GPS, 2 wt% to 20 wt% 1,1-GPS and 30 wt% to 70 wt% 1,1-GPM, or a mixture of 5 wt% to 10 wt% 1,6-GPS, 30 wt% to 40 wt% 1,1-GPS and 45 wt% to 60 wt% 1,1-GPM, or a mixture enriched with 1,6-GPS with a 1,6-GPS content of 57 wt% to 99 wt% and a 1,1-GPM content of 43 wt% to 1 wt% or a mixture enriched with 1,1-GPM with a 1,6-GPS content of 1 wt% to 43 wt% and a 1,1-GPM content of 57 wt% to 99 wt%.

3. Method according to claim 1 or 2, wherein the particle diameter is ≤ 50 µm.

4. Method according to claim 1, 2 or 3, wherein the particle diameter is ≤ 30 µm.

5. Method according to one of the preceding claims, wherein the milling is performed in an air separation ball mill or in a combination of a mill and a downstream air classifier.

6. Method according to one of the preceding claims, wherein additives or auxiliary substances are introduced during milling.

7. Method according to one of the preceding claims, wherein the liquid binder is a solution or suspension of isomalt, a mixture containing 1,6-GPS and 1,1-GPM **characterized by** quantity ratios of 1,1-GPM to 1,6-GPS which differ from those of isomalt, and also containing fat and gelatin or collidone.

8. Method according to one of the preceding claims, wherein the liquid binder is added to the separated ground fraction by spraying.

9. Method according to one of the preceding claims, wherein the liquid binder is added to the separated ground fraction through a nozzle.

10. Method according to one of the preceding claims, wherein agglomeration is performed intermittently in a fluidised-bed agglomerator or in a continuously operated installation.

11. Method according to one of the preceding claims, wherein the liquid binder is added to the separated ground fraction in a form in which it is heated to a temperature above room temperature.

12. Method according to one of the preceding claims, wherein additives and/or flavourings are added to the agglomerate after adding the liquid binder and before pressing.

13. Method according to one of the preceding claims, wherein size fractionation of the agglomerate is performed after adding the liquid binder and before pressing.

14. Method according to one of the preceding claims, wherein the size fractionation of the agglomerate according to claim 13 is performed in a screening machine.

15. Method according to one of the preceding claims, wherein the agglomerate is dried after agglomeration.

16. Compressed product that can be produced according to one of the pr e-ceding claims.

17. Agglomerate that can be produced by process steps a) through c) according to one of claims 1 through 15.

## Revendications

1. Procédé de préparation d'un comprimé comprenant de l'isomaltulose, de l'isomalt ou des mélanges contenant du 1,6-GPS et du 1,1-GPM qui se distinguent par des rapports quantitatifs entre le 1,1-GPM et le 1,6-GPS différents de celui de l'isomalt et/ou contiennent d'autres sucre-alcools, ledit procédé étant **caractérisé en ce que** :
a) l'isomaltulose, l'isomalt et/ou le mélange contenant du 1,6-GPS et du 1,1-GPM sont broyés à sec,
b) une fraction broyée de l'isomaltulose, de l'isomalt ou du mélange contenant du 1,6-GPS et du 1,1-GPM ayant un diamètre maximale de particule 100 µm est obtenue ou séparée soit simultanément soit après ledit broyage à sec,
c) la fraction broyée subit une agglomération par addition d'un agent liant liquide et
d) ladite fraction broyée est ensuite soumise à un processus de pressage pour former un comprimé.

2. Procédé selon la revendication 1, **caractérisé en ce que** le mélange contenant du 1,6-GPS et du 1,1-GPM est un mélange comportant 10 à 50 % en poids de 1,6-GPS, 2 à 20 % en poids de 1,1-GPS et 30 à 70 % en poids de 1,1-GPM, ou un mélange comportant 5 à 10 % en poids de 1,6-GPS, 30 à 40 % en poids de 1.1-GPS et 45 à 60 % en poids de 1,1-GPM, ou un mélange enrichi en 1,6-GPS avec une proportion de 1,6-GPS de 57 à 99 % en poids et une proportion de 1,1-GPM de 43 à 1 % en poids, ou un mélange enrichi en 1,1-GPM avec une proportion de 1,6-GPS de 1 à 43 % en poids et une proportion de 1,1-GPM de 57 à 99 % en poids.

3. Procédé selon la revendication 1 ou 2, **caractérisé en ce que** le diamètre de particule s'élève à ≤ 50 µm.

4. Procédé selon la revendication 1, 2 ou 3, **caractérisé en ce que** le diamètre de particule s'élève à ≤ 30 µm.

5. Procédé selon l'une des revendications précédentes, **caractérisé en ce que** le processus de broyage se fait dans un broyeur-classificateur ou dans une combinaison comprenant un broyeur et un classificateur installé en aval.

6. Procédé selon l'une des revendications précédentes, **caractérisé en ce que** le processus de broyage se fait en ajoutant des adjuvants ou des additifs.

7. Procédé selon l'une des revendications précédentes, **caractérisé en ce que** l'agent liant liquide est une solution ou une suspension de l'isomalt, d'un mélange contenant du 1,6-GPS et du 1,1-GPM qui se distingue par des rapports quantitatifs entre le 1,1-GPM et le 1,6-GPS différents de celui de l'isomalt, de graisse et de gélatine ou de Kollidon.

8. Procédé selon l'une des revendications précédentes, **caractérisé en ce que** l'agent liant liquide est ajouté à la fraction broyée séparée par pulvérisation.

9. Procédé selon l'une des revendications précédentes, **caractérisé en ce que** l'agent liant liquide est ajouté à la fraction broyée séparée via une buse.

10. Procédé selon l'une des revendications précédentes, **caractérisé en ce que** l'agglomération est mise en oeuvre dans un agglomérateur à lit fluidisé en discontinu ou dans un dispositif à action continue.

11. Procédé selon l'une des revendications précédentes, **caractérisé en ce que** l'agent liant liquide est ajouté à la fraction broyée séparée après être chauffé à une température supérieure à la température ambiante.

12. Procédé selon l'une des revendications précédentes, **caractérisé en ce qu'**après l'addition de l'agent liant liquide et avant le processus de pressage, on ajoute des adjuvants et/ou des arômes à l'aggloméré.

13. Procédé selon l'une des revendications précédentes, **caractérisé en ce qu'**après l'addition de l'agent liant liquide et avant le processus de pressage, on met en oeuvre un fractionnement de l'aggloméré en fonction de la dimension.

14. Procédé selon l'une des revendications précédentes, **caractérisé en ce que** le fractionnement de l'aggloméré en fonction de la dimension selon la revendication 13 est mis en oeuvre dans un dispositif à tamiser.

15. Procédé selon l'une des revendications précédentes, **caractérisé en ce que** suite à l'agglomération, on fait sécher l'aggloméré.

16. Comprimé, susceptible d'être produit selon l'une des revendications précédentes.

17. Aggloméré, susceptible d'être produit selon les étapes de procédé a) à c) conformément à l'une des revendications 1 à 15.
